# EUROPÄISCHE PATENTSCHRIFT

(11) **EP 4 497 584 B1**
(45) Veröffentlichungstag und Bekanntmachung des Hinweises auf die Patenterteilung: **27.08.2025**
(21) Anmeldenummer: 24189939.2
(22) Anmeldetag: 22.07.2024
(51) Int. Cl.: B29D 11/00, B29D 11/02, B29C 64/124, B29C 64/245, B33Y 10/00, B33Y 30/00

(54) **VERFAHREN UND ANORDNUNG ZUM HERSTELLEN EINER OPHTHALMOLOGISCHEN VORRICHTUNG**
METHOD AND ARRANGEMENT FOR PRODUCING AN OPHTHALMOLOGICAL DEVICE
PROCÉDÉ ET AGENCEMENT DE FABRICATION D'UN DISPOSITIF OPHTALMOLOGIQUE

(30) Priorität: 26.07.2023 DE 102023207117
(43) Veröffentlichungstag der Anmeldung: 29.01.2025
(73) Patentinhaber: Carl Zeiss Meditec AG, 07745 Jena (DE)
(72) Erfinder: Thaller, Michael, 73447 Oberkochen (DE); Schreiber, Benjamin, 73447 Oberkochen (DE); Badur, Thorben, 73447 Oberkochen (DE); Nicoli, Francesca, 73447 Oberkochen (DE); Masch, Jennifer-Magdalena, 73447 Oberkochen (DE); Srbinoska, Hristina, 73447 Oberkocken (DE)
(74) Vertreter: Patentanwälte Bressel und Partner mbB

(56) Entgegenhaltungen:
- DE-B3- 102020 108 375
- KR-B1- 102 106 102
- US-A1- 2018 001 581
- US-A1- 2020 384 682

## Beschreibung

Die Erfindung betrifft ein Verfahren und eine Anordnung zum Herstellen einer ophthalmologischen Vorrichtung.

Ophthalmologische Vorrichtungen, wie beispielsweise Intraokularlinsen, werden herkömmlich insbesondere durch Drehen hergestellt. Hierzu wird zuerst durch Polymerisation das Ausgangsmaterial hergestellt. Anschließend werden aus dem Ausgangsmaterial Rohlinge geschnitten. Bei hydrophilen Intraokularlinsen werden die Rohlinge werden mittels eines Wachses an einer Drehmaschine befestigt, bei hydrophoben Intraokularlinsen werden die Rohlinge insbesondere bei etwa -20°C angefroren. Ein computergesteuerter Roboterarm, der mit einer Diamantspitze bestückt ist, spant aus dem in der Drehmaschine rotierenden Rohling beispielsweise die Intraokularlinse. Hierbei handelt es sich jedoch um ein aufwändiges und kostenintensives Verfahren. Zudem ist von Nachteil, dass eine beim Drehen verwendete Diamantspitze Rillen auf der Oberfläche der ophthalmologischen Vorrichtung hinterlässt, wobei die Rillen eine optische Qualität der ophthalmologischen Vorrichtung beeinträchtigen.

Aus der DE 10 2020 108 375 B3 ist ein Verfahren zum Herstellen einer Intraokularlinse im Wege des tomographischen Druckens bekannt. Das Verfahren umfasst die Schritte: Bereitstellen eines für elektromagnetische Strahlung transparenten Behälters, in dem eine Flüssigkeit angeordnet ist, die mit der elektromagnetischen Strahlung aushärtbar ist; Bestrahlen der Flüssigkeit mit einem Satz an Bildern, die von der elektromagnetischen Strahlung gebildet werden und die jeweils eine Intraokularlinse zeigen, wobei jedes der Bilder des Satzes unter einem anderen Einstrahlwinkel bezüglich einer durch die Flüssigkeit verlaufenden Bezugsebene in die Flüssigkeit eingestrahlt wird, wodurch die Flüssigkeit ausgehärtet wird und die ausgehärtete Flüssigkeit die Intraokularlinse bildet, wobei in der Flüssigkeit ein Aktuator, ein Solarmodul und/oder ein Sensor angeordnet ist und die Intraokularlinse um den Aktuator, das Solarmodul und/oder den Sensor herum gebildet wird.

Aus der WO 2019/043529 A1 ist ein Verfahren zum Herstellen eines dreidimensionalen Objekts bekannt, umfassend das Berechnen einer Folge von Rückprojektionen, die das zu bildende dreidimensionale Objekt beschreiben, aus verschiedenen Orientierungswinkeln des Objekts, Definieren einer Folge von Lichtmustern unter Verwendung der Rückprojektionen und Bestrahlen eines photoresponsiven Materials, das in der Lage ist, seine materielle Phase bei Bestrahlung mit Licht zu ändern mit jedem der Lichtmuster bei dem jeweiligen entsprechenden Orientierungswinkel und gemäß der definierten Sequenz, wodurch eine dreidimensionale Verteilung von Änderungen innerhalb des photoresponsiven Mediums erzeugt wird, die das dreidimensionale Objekt physikalisch reproduziert, wodurch das dreidimensionale Objekt erzeugt wird.

Der Erfindung liegt die Aufgabe zu Grunde, ein Verfahren und eine Anordnung zum Herstellen einer ophthalmologischen Vorrichtung zu verbessern.

Die Aufgabe wird erfindungsgemäß durch ein Verfahren mit den Merkmalen des Patentanspruchs 1 und eine Anordnung mit den Merkmalen des Patentanspruchs 12 gelöst. Vorteilhafte Ausgestaltungen der Erfindung ergeben sich aus den Unteransprüchen.

Es ist einer der Grundgedanken der Erfindung, eine ophthalmologische Vorrichtung mit mindestens einem ersten Teilstück und mindestens einem zweiten Teilstück herzustellen. Das mindestens eine erste Teilstück wird hierbei bereitgestellt, das mindestens eine zweite Teilstück wird hingegen tomographisch gedruckt. Hierdurch lässt sich die ophthalmologische Vorrichtung derart herstellen, dass zumindest ein zweites Teilstück an dem mindestens einen ersten Teilstück angeordnet ist und/oder dieses zumindest teilweise umschließt. Hierdurch kann das mindestens eine erste Teilstück zumindest teilweise in das mindestens eine zweite Teilstück integriert werden. Hierzu ist vorgesehen, dass das mindestens eine erste Teilstück (bereits fertig) bereitgestellt wird, wobei das mindestens eine erste Teilstück grundsätzlich mittels eines beliebigen Herstellungsverfahrens hergestellt werden kann. Das mindestens eine erste Teilstück wird mindestens einer tomographischen Druckeinrichtung zugeführt. Dies erfolgt mittels einer laminaren Strömung in einer aushärtbaren Flüssigkeit durch ein für elektromagnetische Strahlung transparentes Rohr hindurch. Das mindestens eine zweite Teilstück der ophthalmologischen Vorrichtung wird anschließend in der mindestens einen tomographischen Druckeinrichtung tomographisch gedruckt. Hierzu wird für das mindestens eine zweite Teilstück ein Datensatz aus Bildern des zweiten Teilstücks der ophthalmologischen Vorrichtung erzeugt und/oder bereitgestellt, wobei die Bilder Projektionen dieses mindestens einen zweiten Teilstücks aus unterschiedlichen Richtungen beinhalten. Die in dem transparenten Rohr enthaltene mittels elektromagnetischer Strahlung aushärtbare Flüssigkeit wird dann ausgehend von dem erzeugten und/oder bereitgestellten Datensatz zum Ausbilden des mindestens einen zweiten Teilstücks tomographisch gedruckt, das heißt, insbesondere in Abhängigkeit von den Lichtmustern in den Bildern des Datensatzes lokal ausgehärtet. Das Erzeugen und/oder Bereitstellen des Datensatzes und das tomographische Drucken erfolgen hierbei derart, dass das mindestens eine zweite Teilstück an dem mindestens einen ersten Teilstück angeordnet ist und/oder dieses zumindest teilweise umschließt. Nach dem tomographischen Drucken des mindestens einen zweiten Teilstücks wird das mindestens eine erste Teilstück und das hieran angeordnete mindestens eine zweite Teilstück mittels einer laminaren Strömung in der aushärtbaren Flüssigkeit von der mindestens einen tomographischen Druckeinrichtung abgeführt.

Insbesondere wird ein Verfahren zum Herstellen einer ophthalmologischen Vorrichtung zur Verfügung gestellt, umfassend: Bereitstellen eines für elektromagnetische Strahlung transparenten Rohres, Befüllen des transparenten Rohres mit einer mittels elektromagnetischer Strahlung aushärtbaren Flüssigkeit, Einbringen von mindestens einem ersten Teilstück der ophthalmologischen Vorrichtung in das transparente Rohr, Zuführen des mindestens einen ersten Teilstücks mittels einer laminaren Strömung in der aushärtbaren Flüssigkeit zu mindestens einer tomographischen Druckeinrichtung, Erzeugen und/oder Bereitstellen eines Datensatzes aus Bildern mindestens eines zweiten Teilstücks der ophthalmologischen Vorrichtung, wobei die Bilder Projektionen des mindestens einen zweiten Teilstücks aus unterschiedlichen Richtungen beinhalten, tomographisches Drucken der aushärtbaren Flüssigkeit in dem transparenten Rohr mittels elektromagnetischer Strahlung ausgehend von dem erzeugten und/oder bereitgestellten Datensatz zum Ausbilden des mindestens einen zweiten Teilstücks mittels der mindestens einen tomographischen Druckeinrichtung, wobei das Erzeugen und/oder Bereitstellen des Datensatzes und das tomographische Drucken derart erfolgen, dass das mindestens eine zweite Teilstück an dem mindestens einen ersten Teilstück angeordnet ist und/oder dieses zumindest teilweise umschließt, und Abführen des mindestens einen ersten Teilstücks und des hieran angeordneten mindestens einen zweiten Teilstücks mittels einer laminaren Strömung in der aushärtbaren Flüssigkeit von der mindestens einen tomographischen Druckeinrichtung.

Ferner wird insbesondere eine Anordnung zum Herstellen einer ophthalmologischen Vorrichtung geschaffen, umfassend ein für elektromagnetische Strahlung transparentes Rohr, eine Strömungserzeugungseinrichtung, die eingerichtet ist zum Erzeugen einer laminaren Strömung in einer in dem transparenten Rohr enthaltenen mittels elektromagnetischer Strahlung aushärtbaren Flüssigkeit, eine Datenverarbeitungseinrichtung und mindestens eine tomographische Druckeinrichtung, wobei die Strömungserzeugungseinrichtung ferner dazu eingerichtet ist und das transparente Rohr derart angeordnet ist, mindestens ein erstes Teilstück der ophthalmologischen Vorrichtung mittels der erzeugten laminaren Strömung zu der mindestens einen tomographischen Druckeinrichtung zuzuführen und wieder von dieser abzuführen, wobei die Datenverarbeitungseinrichtung dazu eingerichtet ist, einen Datensatz aus Bildern mindestens eines zweiten Teilstücks der ophthalmologischen Vorrichtung zu erzeugen und/oder bereitzustellen, wobei die Bilder Projektionen dieses mindestens einen zweiten Teilstücks aus unterschiedlichen Richtungen beinhalten, wobei die tomographische Druckeinrichtung dazu eingerichtet ist, die aushärtbare Flüssigkeit in dem transparenten Rohr mittels elektromagnetischer Strahlung ausgehend von dem erzeugten und/oder bereitgestellten Datensatz zum Ausbilden des mindestens einen zweiten Teilstücks tomographisch zu drucken, wobei das Erzeugen und/oder Bereitstellen des Datensatzes und das tomographische Drucken derart erfolgen, dass das mindestens eine zweite Teilstück an dem mindestens einen ersten Teilstück angeordnet ist und/oder dieses zumindest teilweise umschließt.

Ein Vorteil des Verfahrens und der Anordnung ist, dass eine Vielzahl von ophthalmologischen Vorrichtungen in einer Art Fließbandproduktion hergestellt werden kann. Hierdurch kann beim Herstellen ein Durchsatz erhöht werden, sodass insgesamt Aufwand und Kosten eingespart werden können. Hierzu kann beispielsweise vorgesehen sein, eine Vielzahl von den ersten Teilstücken mittels der laminaren Strömung zu bewegen und der tomographischen Druckeinrichtung zuzuführen und wieder von dieser abzuführen. Hierbei kann insbesondere vorgesehen sein, dass eine Vielzahl gleichzeitig tomographisch gedruckt wird, wobei dies mittels einer oder mittels mehrerer tomographischer Druckeinrichtungen erfolgen kann. Alternativ kann vorgesehen sein, dass jedes erste Teilstück einzeln in die mindestens eine tomographische Druckeinrichtung befördert wird und das mindestens eine zweite Teilstück jeweils für jede ophthalmologische Vorrichtung einzeln tomographisch gedruckt wird.

Ein weiterer Vorteil des Verfahrens und der Anordnung ist, dass prinzipiell beliebige Materialien (Werkstoffe) miteinander kombiniert werden können. Beispielsweise kann vorgesehen sein, dass das erste Teilstück eine Haptik einer Intraokularlinse ist und das zweite Teilstück eine Optik der Intraokularlinse ausbildet. Ein Material (Werkstoff) des ersten Teilstücks kann hierbei grundsätzlich nahezu beliebig gewählt sein und/oder auf beliebige Weise hergestellt werden.

Die elektromagnetische Strahlung liegt insbesondere im optischen Wellenlängenbereich, insbesondere im sichtbaren und/oder UV-Wellenlängenbereich. Die aushärtbare Flüssigkeit kann insbesondere die Eigenschaften aufweisen, welche in der DE 10 2020 108 375 B3 beschrieben sind. Insbesondere erfolgt das tomographische Drucken grundsätzlich auf die in der DE 10 2020 108 375 B3 beschriebene Art und Weise. Die Bilder des Datensatzes können zum Erzeugen und/oder Bereitstellen beispielsweise aus einem dreidimensionalen Datensatz (z.B. CAD-Daten), der die Form der ophthalmologischen Vorrichtung, insbesondere des mindestens einen zweiten Teilstücks, beinhaltet, errechnet werden. Hierbei handelt es sich insbesondere um einen umgekehrten Prozess, wie er bei einer tomographischen Bildgebung eingesetzt wird. Die tomographische Bildgebung kommt beispielsweise bei einer Computertomographie zum Einsatz. Die tomographische Bildgebung kann beispielsweise von einer Radon-Transformation Gebrauch machen. Das tomographische Drucken macht sich insbesondere diesen umgekehrten Prozess zunutze, um mittels der errechneten Bilder die aushärtbare Flüssigkeit in Abhängigkeit von den in den Bildern des Datensatzes enthaltenen Lichtmustern lokal auszuhärten.

Die aushärtbare Flüssigkeit umfasst insbesondere eine Lösung mit einem gelösten Monomer und einem Fotoinitiator, welcher strahlungsabhängig eine Polymerisation des Monomers auslösen kann. Ferner können auch weitere Stoffe Bestandteil der aushärtbaren Flüssigkeit sein, wie z.B. Füllstoffe, optisch anregbare Farbstoffe oder Nanopartikel und/oder medizinische Wirkstoffe. Insbesondere ist vorgesehen, dass die aushärtbare Flüssigkeit eine vorgegebene Mindestviskosität von mindestens 100 mPa*s (cps) aufweist.

Es kann vorgesehen sein, dass die ophthalmologische Vorrichtung eine Intraokularlinse ist. Die Intraokularlinse kann auch eine akkommodierende Intraokularlinse sein. Die ophthalmologische Vorrichtung, insbesondere die Intraokularlinse, kann als erstes Teilstück insbesondere auch einen Aktuator, ein Solarmodul und/oder einen Sensor aufweisen. Ferner kann die ophthalmologische Vorrichtung, insbesondere die Intraokularlinse, einen Optikkörper und mindestens eine Haptik umfassen.

Die ophthalmologische Vorrichtung kann ferner eine der folgenden sein: eine Glaukom-Drainagevorrichtung, ein Augenstent, ein chirurgischer Port, ein Kapselring (engl. capsular ring), ein Kapselspannring (engl. capsular tension ring), ein Augapfelring, eine Kapselstützvorrichtung, ein Hornhautimplantat, ein Iris-Implantat (z.B. mit Farbstoffpartikeln), eine Iris-Prothese, eine Kontaktlinse, eine therapeutische Kontaktlinse mit einem medizinischen Wirkstoff, eine implantierbare Kontaktlinse, ein Sattelring, ein Iris-Expanderring usw.

Es kann vorgesehen sein, dass die verfahrensgemäß hergestellte ophthalmologische Vorrichtung anschließend noch nachbearbeitet wird, beispielsweise durch Drehen, mechanisches Polieren, Laserpolieren und/oder Laserschneiden etc.

Teile der Anordnung, insbesondere die Datenverarbeitungseinrichtung, können einzeln oder zusammengefasst als eine Kombination von Hardware und Software ausgebildet sein, beispielsweise als Programmcode, der auf einem Mikrocontroller oder Mikroprozessor ausgeführt wird. Es kann jedoch auch vorgesehen sein, dass Teile einzeln oder zusammengefasst als anwendungsspezifische integrierte Schaltung (ASIC) und/oder feldprogrammierbares Gatterfeld (FPGA) ausgebildet sind.

In einer Ausführungsform ist vorgesehen, dass die aushärtbare Flüssigkeit einen vorgegebenen Anteil an Kieselgel umfasst. Der Anteil kann beispielsweise sein wie folgt, ist aber nicht beschränkt auf diese Werte: 5% (w/w), 10% (w/w), 15% (w/w), 20% (w/w), 25% (w/w), 30% (w/w), 35% (w/w), 40% (w/w), 45% (w/w), 50% (w/w), 55% (w/w), 60% (w/w). Hierdurch kann eine Viskosität eingestellt werden. Beispielsweise kann vorgesehen sein, als aushärtbare Flüssigkeit Methylmethacrylat gemischt mit einem Fotoinitiator, einem Vernetzer (z.B. Trimethylolpropantrimethactylat) und dem Kieselgel als Verdickungsmittel zu verwenden. Als Kieselgel kann beispielsweise CAB-O-SIL^{®} der Firma Cabot Corporation, USA, verwendet werden.

In einer Ausführungsform ist vorgesehen, dass die aushärtbare Flüssigkeit vorpolymerisiertes Methylmethacrylat und/oder eine Mischung aus langkettigen Polymeren und kurzen Monomeren umfasst. Hierdurch kann eine Viskosität eingestellt und insbesondere erhöht werden. Beispielsweise kann vorgesehen sein, in der Mischung teilweise vorpolymerisiertes Methylmethacrylat zu verwenden, sodass teilweise längere Ketten von Polymethylmethacrylat (PMMA) vorliegen, welche eine Viskosität der aushärtbaren Flüssigkeit erhöhen. Insbesondere ist hierbei vorgesehen, eine vollständige Polymerisierung zu verhindern. Eine vollständige Polymerisierung wird dann erst durch das tomographische Drucken ausgelöst. Entsprechendes gilt für die Mischung aus langkettigen Polymeren und kurzen Monomeren. Beispielsweise kann langkettiges Poly(ethylenglycol)acrylat gemischt mit kurzen Monomeren verwendet werden. In der nachfolgenden Tabelle 1 sind ein paar Beispiele aufgeführt, welche repräsentativ, aber nicht einschränkend sind. Die Menge an möglichen Monomeren, Präpolymeren, Kombinationen aus verschiedenen Ausgangsstoffen und individuellen Monomeren ist derart groß, dass nur eine kleine Auswahl aufgelistet ist, ohne dass diese Auswahl einschränkend sein soll. Z.B. kann eines der PMMA-Präpolymere mit einem oder mehreren der sekundären Monomere in der Tabelle 1 verbunden werden. Es ist auch möglich, verschiedene Präpolymere zu mischen (z.B. PMMA-Präpolymere verschiedener Längen, oder Präpolymere aus PMMA mit Präpolymeren der sekundären Monomere). Weiterhin kann der Mischungsanteil mit jedem möglichen Verhältnis zueinander variiert werden, um gewünschte Eigenschaften des resultierenden Copolymers zu erhalten.

**Tabelle 1: Beispiele für Formulierungen aus PMMA-Präpolymeren und verschiedenen Kandidaten für Copolymere. Dabei kann jedes der Präpolymere mit jedem der sekundären Monomere verknüpft werden. Auch die jeweiligen Mengenanteile können variabel gewählt werden.**

| **PMMA-Präpolymer** | **Sekundäres Monomer** | **Crosslinker** |
|---|---|---|
| (C₅O₂H₈)₅₀₋₁₀₀ | Hydroxyethylmethacrylat | Trimethylopropantrimethacrylat |
| (C₅O₂H₈)₁₀₀₋₂₀₀ | Ethoxyethylmethacrylat | |
| (C₅O₂H₈)₅₀₀₋₇₀₀ | Ethylenglycoldimethacrylat | |
| (C₅O₂H₈)₈₀₀₋₁₀₀₀ | Butylacrylat | |
| (C₅O₂H₈)₁₀₀₀₋₁₅₀₀ | Ethylmethacrylat | |

In einer Ausführungsform ist vorgesehen, dass das transparente Rohr zusammen mit der hierin enthaltenen aushärtbaren Flüssigkeit zumindest während des tomographischen Druckens um eine Längsachse gedreht wird. Hierdurch kann mittels einer einzigen Belichtungseinrichtung der tomographischen Druckeinrichtung aus jeder Richtung das jeweilige Bild des Datensatzes in die aushärtbare Flüssigkeit projiziert werden. Hierbei wird die Viskosität der aushärtbaren Flüssigkeit insbesondere so groß gewählt, dass das mindestens eine erste Teilstück beim Drehen der aushärtbaren Flüssigkeit zusammen mit der aushärtbaren Flüssigkeit mitgedreht wird. Es kann vorgesehen sein, dass ein Starten und ein Beenden der Drehung mittels einer Drehgeschwindigkeitsrampe erfolgen, sodass eine Geschwindigkeitsänderung der Drehung nur klein ist und sichergestellt ist, dass das erste Teilstück sich mitdrehen kann.

In einer Ausführungsform ist vorgesehen, dass die aushärtbare Flüssigkeit zumindest abschnittsweise mittels einer Kühleinrichtung gekühlt wird. Hierdurch kann eine Viskosität der aushärtbaren Flüssigkeit zumindest abschnittsweise erhöht werden. Insbesondere kann vorgesehen sein, eine Temperatur der aushärtbaren Flüssigkeit zumindest während des tomographischen Druckens zu verringern, um ein schwerkraftgetriebenes Absinken des mindestens einen ersten Teilstücks innerhalb der aushärtbaren Flüssigkeit während des tomographischen Druckens zu verhindern oder zumindest zu verringern.

In einer Ausführungsform ist vorgesehen, dass das tomographische Drucken des jeweiligen mindestens einen zweiten Teilstücks für mehrere ophthalmologische Vorrichtungen gleichzeitig erfolgt. Hierdurch kann ein Durchsatz bei der hergestellten Stückzahl von ophthalmologischen Vorrichtungen erhöht werden, wodurch sich Kosten und Aufwand reduzieren lassen. Es kann vorgesehen sein, dass hierzu mehrere erste Teilstücke in einer tomographischen Druckeinrichtung angeordnet und dort gleichzeitig tomographisch gedruckt werden, wobei die Bilder des Datensatzes hierfür für mehrere zweite Teilstücke entsprechend erzeugt und/oder bereitgestellt werden. Es können jedoch auch mehrere tomographische Druckeinrichtungen vorgesehen sein, in denen jeweils ein erstes Teilstück oder mehrere erste Teilstücke zum gleichzeitigen tomographischen Drucken der jeweiligen zweiten Teilstücke angeordnet werden.

In einer Ausführungsform ist vorgesehen, dass eine Position des mindestens einen ersten Teilstücks in der aushärtbaren Flüssigkeit mittels mindestens einer von außen in die aushärtbare Flüssigkeit eingespeisten Energie und/oder Kraft stabilisiert und/oder korrigiert wird. Hierdurch kann insbesondere ein schwerkraftgetriebenes Absinken des mindestens einen ersten Teilstücks in der aushärtbaren Flüssigkeit verringert oder sogar verhindert werden. Das Korrigieren und/oder Stabilisieren kann vor dem tomographischen Drucken erfolgen. Es ist jedoch alternativ oder zusätzlich auch möglich, das Stabilisieren und/oder Korrigieren während des tomographischen Druckens durchzuführen. Hierbei ist jedoch zu beachten, dass das Stabilisieren und/oder Korrigieren das tomographische Drucken nicht beeinträchtigt. Es kann vorgesehen sein, dass eine Einrichtung zum Korrigieren und/oder Stabilisieren parallel zu dem transparenten Rohr mit einem oder mehreren ersten Teilstücken mitbewegt wird. Hierzu kann beispielsweise eine Schiene und/oder ein Linearantrieb vorgesehen sein. Auch eine Drehbewegung des transparenten Rohres um die Längsachse kann beim Stabilisieren und/oder Korrigieren berücksichtigt werden. Es kann vorgesehen sein, dass eine Position und/oder Orientierung des mindestens einen ersten Teilstücks mittels mindestens einer Sensorik erfasst wird, wobei das Stabilisieren und/oder Korrigieren ausgehend von der erfassten Position und/oder Orientierung erfolgt. Beispielsweise kann eine Differenz zwischen einer erfassten IstPosition und/oder Ist-Orientierung und einer Soll-Position bzw. Soll-Orientierung bestimmt werden und das Stabilisieren und/oder Korrigieren ausgehend von der bestimmten Differenz erfolgen.

In einer Ausführungsform ist vorgesehen, dass eine Position des mindestens einen ersten Teilstücks in der aushärtbaren Flüssigkeit mittels mindestens einer optischen Pinzette stabilisiert und/oder korrigiert wird. Hierbei ist insbesondere vorgesehen, dass eine von der optischen Pinzette verwendete Wellenlänge nicht das tomographische Drucken beeinträchtigt.

In einer Ausführungsform ist vorgesehen, dass eine Position des mindestens einen ersten Teilstücks in der aushärtbaren Flüssigkeit mittels mindestens eines elektrischen Feldes und/oder mindestens eines magnetischen Feldes stabilisiert und/oder korrigiert wird. Dies ist möglich, wenn das erste Teilstück mittels elektrischer und/oder magnetischer Felder beeinflusst werden kann. Insbesondere können hierzu gezielt gerichtete und/oder fokussierte elektrische und/oder magnetische Felder erzeugt werden. Beinhaltet das erste Teilstück beispielsweise ferromagnetische Bereiche, so kann das erste Teilstück mittels eines solchen magnetischen Feldes in Position gehalten werden.

In einer Ausführungsform ist vorgesehen, dass eine Position des mindestens einen ersten Teilstücks in der aushärtbaren Flüssigkeit mittels akusto-mechanischen Kräften stabilisiert und/oder korrigiert wird. Insbesondere kann dies mittels stehender akustischer Wellen erreicht werden. Mittels der stehenden Wellen können Kräfte auf das erste Teilstück ausgeübt werden und eine Position des ersten Teilstücks kann hierüber stabilisiert und/oder korrigiert werden. Als Grundlage kann beispielsweise das in Adem Ozcelik et al., Acoustic tweezers for the life sciences, Nature Methods, Vol. 15, Ausgabe 12, S. 1021-1028, 26. Nov. 2018, https://doi.org/10.1038/s41592-018-0222-9, beschriebene Verfahren dienen.

In einer Ausführungsform ist vorgesehen, dass eine Position des mindestens einen ersten Teilstücks in der aushärtbaren Flüssigkeit mittels einer entgegen der Schwerkraft ausgerichteten laminaren Strömung stabilisiert und/oder korrigiert wird. Hierdurch kann das mindestens eine erste Teilstück in Position gehalten werden. Diese Ausführungsform kann insbesondere eingesetzt werden, wenn die Viskosität der aushärtbaren Flüssigkeit nicht ausreichend groß ist, um das mindestens eine erste Teilstück in Position zu halten. Durch Erzeugen einer laminaren Strömung, welche entgegen der Richtung der Schwerkraft wirkt, kann das schwerkraftgetriebene Absinken des mindestens einen ersten Teilstücks kompensiert werden. Eine Geschwindigkeit der entgegengesetzt zur Schwerkraft wirkenden laminaren Strömung wird hierbei entsprechend einer Absinkgeschwindigkeit des mindestens einen ersten Teilstücks gewählt, sodass das mindestens eine erste Teilstück in der Summe an derselben Position gehalten wird. Es kann vorgesehen sein, dass eine Position des mindestens einen ersten Teilstücks mittels mindestens einer Sensorik erfasst wird, wobei eine Geschwindigkeit der laminaren Strömung, welche der Schwerkraft entgegengesetzt wirkt, ausgehend von der erfassten Position gewählt wird. Beispielsweise kann aus einer Änderung der erfassten Position eine Absinkgeschwindigkeit bestimmt werden und eine Geschwindigkeit der laminaren Strömung, welche entgegen der Schwerkraft wirkt, ausgehend von der bestimmten Geschwindigkeit gewählt und eingestellt werden. Insbesondere wird die Geschwindigkeit dem Betrag nach gleich gewählt, jedoch mit einem umgekehrten Vorzeichen, sodass eine Richtung der laminaren Strömung entgegensetzt zur Schwerkraft ist.

Weitere Merkmale zur Ausgestaltung der Anordnung ergeben sich aus der Beschreibung von Ausgestaltungen des Verfahrens. Die Vorteile der Anordnung sind hierbei jeweils die gleichen wie bei den Ausgestaltungen des Verfahrens.

Nachfolgend wird die Erfindung anhand bevorzugter Ausführungsbeispiele unter Bezugnahme auf die Figuren näher erläutert. Hierbei zeigen:
- Fig. 1: ein schematisches Ablaufdiagramm von Ausführungsformen des Verfahrens zum Herstellen einer ophthalmologischen Vorrichtung;
- Fig. 2: eine schematische Darstellung zur Verdeutlichung einer Ausführungsform des Verfahrens;
- Fig. 3: eine schematische Darstellung zur Verdeutlichung von Ausführungsformen der Anordnung.

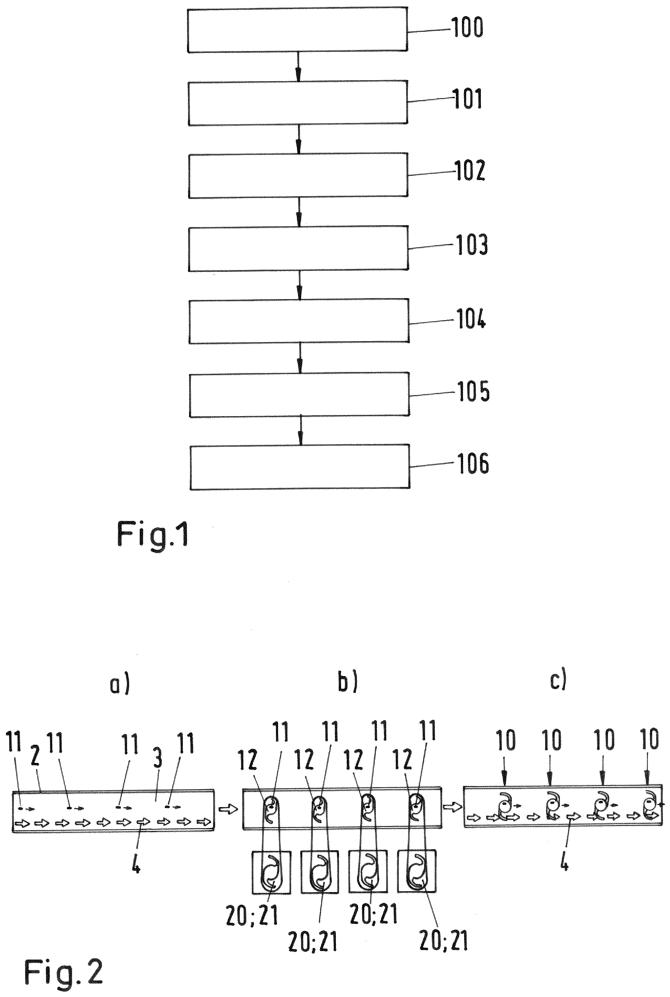

Die Fig. 1 zeigt ein schematisches Ablaufdiagramm von Ausführungsformen des Verfahrens zum Herstellen einer ophthalmologischen Vorrichtung. Es wird nachfolgend beispielhaft davon ausgegangen, dass eine ophthalmologische Vorrichtung ein erstes Teilstück und ein zweites Teilstück umfasst. Grundsätzlich kann die ophthalmologische Vorrichtung auch mehrere erste Teilstücke und/oder mehrere zweite Teilstücke umfassen. Die ophthalmologische Vorrichtung ist beispielsweise eine Intraokularlinse, wobei das erste Teilstück eine Haptik und das zweite Teilstück eine Optik ist. Die Optik soll tomographisch um einen Teil der Haptik herum gedruckt werden.

In einem Verfahrensschritt 100 wird ein für elektromagnetische Strahlung transparentes Rohr bereitgestellt. Das Rohr verläuft hierbei insbesondere horizontal. Grundsätzlich kann das Rohr aber auch in einer anderen Richtung verlaufen, beispielsweise vertikal.

In einem Verfahrensschritt 101 wird das transparente Rohr mit einer mittels elektromagnetischer Strahlung aushärtbaren Flüssigkeit befüllt. Die aushärtbare Flüssigkeit weist hierbei insbesondere eine vorgegebene Viskosität auf.

In einem Verfahrensschritt 102 wird das erste Teilstück der ophthalmologischen Vorrichtung in das transparente Rohr eingebracht. Das erste Teilstück wird hierbei insbesondere mittig mit Bezug auf eine Querschnittsfläche des Rohres angeordnet. Insbesondere werden mehrere erste Teilstücke, das heißt, jeweils ein erstes Teilstück für mehrere ophthalmologische Vorrichtungen, in das transparente Rohr eingebracht. Es kann vorgesehen sein, dass hierzu eine Schleuse vorgesehen ist, durch die das erste Teilstück (bzw. die mehreren ersten Teilstücke) in das Rohr in die aushärtbare Flüssigkeit eingebracht werden können.

In einem Verfahrensschritt 103 wird das erste Teilstück mittels einer laminaren Strömung in der aushärtbaren Flüssigkeit mindestens einer tomographischen Druckeinrichtung zugeführt. Die laminare Strömung wird hierzu mittels einer Strömungserzeugungseinrichtung erzeugt. Es ist hierbei insbesondere vorgesehen, dass eine Strömungsgeschwindigkeit zum Aufbauen der laminaren Strömung langsam geändert wird, sodass das in der aushärtbaren Flüssigkeit schwebende erste Teilstück mit dieser mitbewegt wird und eine Relativposition zu der (bewegten) aushärtbaren Flüssigkeit nicht ändert, sondern von dieser mitgetragen wird.

In einem Verfahrensschritt 104 wird ein Datensatz aus Bildern des zweiten Teilstücks der ophthalmologischen Vorrichtung erzeugt und/oder bereitgestellt, wobei die Bilder Projektionen des zweiten Teilstücks aus unterschiedlichen Richtungen beinhalten.

In einem Verfahrensschritt 105 wird die aushärtbare Flüssigkeit in dem transparenten Rohr mittels elektromagnetischer Strahlung ausgehend von dem erzeugten und/oder bereitgestellten Datensatz zum Ausbilden des zweiten Teilstücks mittels der mindestens einen tomographischen Druckeinrichtung tomographisch gedruckt. Es kann hierbei vorgesehen sein, dass hierzu eine Geschwindigkeit der laminaren Strömung reduziert oder sogar auf Null gesetzt wird, sodass das erste Teilstück beim tomographischen Drucken relativ zur tomographischen Druckeinrichtung langsamer oder überhaupt nicht bewegt wird.

Das Erzeugen und/oder Bereitstellen des Datensatzes in Verfahrensschritt 104 und das tomographische Drucken in Verfahrensschritt 105 erfolgen derart, dass das zweite Teilstück an dem ersten Teilstück angeordnet ist und/oder dieses zumindest teilweise umschließt.

In einem Verfahrensschritt 106 wird das erste Teilstück und das hieran angeordnete zweite Teilstück mittels einer laminaren Strömung in der aushärtbaren Flüssigkeit von der mindestens einen tomographischen Druckeinrichtung abgeführt.

Es kann vorgesehen sein, dass weitere Stationen vorhanden sind, zu denen die bereits hergestellte ophthalmologische Vorrichtung mittels der laminaren Strömung transportiert wird.

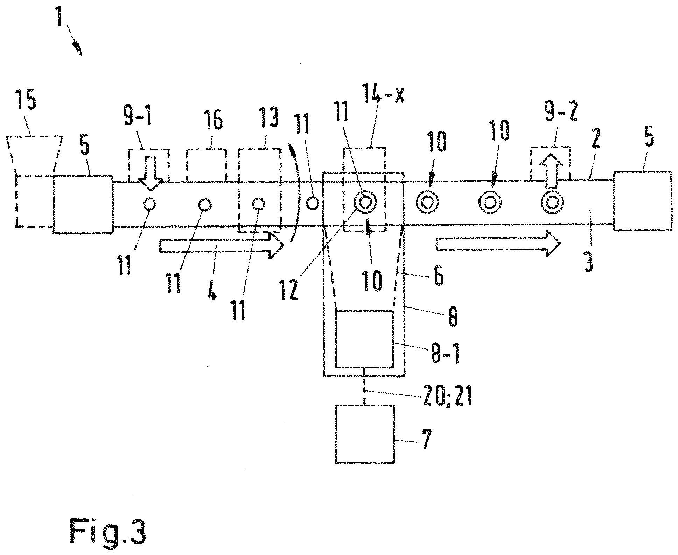

Weitere Ausführungsformen des Verfahrens ergeben sich aus den nachfolgend mit Bezug auf die Figuren 2 und 3 beschriebenen Ausführungsformen.

Die Fig. 2 zeigt eine schematische Darstellung mit den Schritten a) bis c) zur Verdeutlichung des Verfahrens. Im Schritt a) werden die mittig in dem transparenten Rohr 2 in der aushärtbaren Flüssigkeit 3 angeordneten ersten Teilstücke 11 mittels der laminaren Strömung 4 zur mindestens einen tomographischen Druckeinrichtung transportiert. Die ersten Teilstücke 11 sind hierbei insbesondere in gleichen Abständen zueinander angeordnet. Im Schritt b) werden mehrere zweite Teilstücke 12 tomographisch derart gedruckt, dass die zweiten Teilstücke 12 jeweils an dem ersten Teilstück 11 angeordnet sind und/oder diese jeweils zumindest teilweise umschließen. In Schritt c) werden die fertigen ophthalmologischen Vorrichtungen 10, welche sowohl das erste Teilstück 11 als auch das hieran angeordnete zweite Teilstück 12 umfassen, mittels einer laminaren Strömung 4 abgeführt und hierdurch insbesondere von der tomographischen Druckeinrichtung abtransportiert.

Die Fig. 3 zeigt eine schematische Darstellung von Ausführungsformen der Anordnung 1 zum Herstellen einer ophthalmologischen Vorrichtung 10. Die Anordnung 1 umfasst ein für elektromagnetische Strahlung 6 transparentes Rohr 2, eine Strömungserzeugungseinrichtung 5, die eingerichtet ist zum Erzeugen einer laminaren Strömung 4 in einer in dem transparenten Rohr 2 enthaltenen mittels elektromagnetischer Strahlung 6 aushärtbaren Flüssigkeit 3, eine Datenverarbeitungseinrichtung 7, welche beispielsweise eine Recheneinrichtung und einen Speicher umfasst (beide nicht gezeigt) und eine tomographische Druckeinrichtung 8.

Ferner umfasst die Anordnung 1 insbesondere eine Schleuse 9-1 zum Einschleusen von ersten Teilstücken 11 in das Rohr 2 und eine Schleuse 9-2 zum Ausschleusen der fertigen ophthalmologischen Vorrichtungen 10 aus dem Rohr 2. Die Anordnung 1 kann weiter auch eine Befülleinrichtung 15 aufweisen, welche zum Befüllen des Rohres 2 mit der aushärtbaren Flüssigkeit 3 eingerichtet ist.

Die Strömungserzeugungseinrichtung 5 ist dazu eingerichtet und das transparente Rohr 2 ist derart angeordnet, die ersten Teilstücke 11 der ophthalmologischen Vorrichtung 10 mittels der erzeugten laminaren Strömung 4 der tomographischen Druckeinrichtung 8 zuzuführen und nach dem tomographischen Drucken wieder von dieser abzuführen.

Die Datenverarbeitungseinrichtung 7 ist dazu eingerichtet, einen Datensatz 20 aus Bildern 21 eines zweiten Teilstücks 12 der ophthalmologischen Vorrichtung 10 zu erzeugen und/oder bereitzustellen, wobei die Bilder 21 Projektionen des zweiten Teilstücks 12 aus unterschiedlichen Richtungen beinhalten.

Die tomographische Druckeinrichtung 8 ist dazu eingerichtet, die aushärtbare Flüssigkeit 3 in dem transparenten Rohr 2 mittels elektromagnetischer Strahlung 6 ausgehend von dem erzeugten und/oder bereitgestellten Datensatz 20 zum Ausbilden des zweiten Teilstücks 12 tomographisch zu drucken. Hierzu werden die Bilder 21 im Datensatz 20 mittels einer Belichtungseinrichtung 8-1 der tomographischen Druckeinrichtung 8 aus unterschiedlichen Richtungen in die aushärtbare Flüssigkeit 3 projiziert, sodass die aushärtbare Flüssigkeit 3 gezielt aushärtet und den zweiten Teilbereich 12 ausbildet.

Es kann hierzu vorgesehen sein, dass das transparente Rohr 2 zusammen mit der hierin enthaltenen aushärtbaren Flüssigkeit 3 zumindest während des tomographischen Druckens um eine Längsachse gedreht wird, wie dies durch den Pfeil angedeutet ist. Die Anordnung 1 kann hierzu eine Dreheinrichtung 16 aufweisen. Alternativ kann auch vorgesehen sein, dass die Belichtungseinrichtung 8-1 um das transparente Rohr 2 herum gedreht wird, um aus unterschiedlichen Richtungen zu bestrahlen bzw. zu belichten. Es kann alternativ oder zusätzlich auch vorgesehen sein, dass die tomographische Druckeinrichtung 8 mehr als eine Belichtungseinrichtung 8-1 aufweist, sodass gleichzeitig aus mehreren Richtungen belichtet werden kann.

Das Erzeugen und/oder Bereitstellen des Datensatzes 20 und das tomographische Drucken erfolgen derart, dass das zweite Teilstück 12 an dem ersten Teilstück 11 angeordnet ist und/oder dieses zumindest teilweise umschließt, wie dies in der Fig. 3 schematisch angedeutet ist.

Es kann vorgesehen sein, dass die aushärtbare Flüssigkeit 3 einen vorgegebenen Anteil an Kieselgel umfasst.

Es kann vorgesehen sein, dass die aushärtbare Flüssigkeit 3 vorpolymerisiertes Methylmethacrylat und/oder eine Mischung aus langkettigen Polymeren und kurzen Monomeren umfasst.

Es kann vorgesehen sein, dass die aushärtbare Flüssigkeit 3 zumindest abschnittsweise mittels einer Kühleinrichtung 13 gekühlt wird. Die Anordnung 1 weist hierzu eine Kühleinrichtung 13 auf, welche die aushärtbare Flüssigkeit 3 herunterkühlt, sodass eine Viskosität ansteigt. Die Kühleinrichtung 13 ist beispielsweise direkt vor der tomographischen Druckeinrichtung 8 angeordnet.

Es kann vorgesehen sein, dass das tomographische Drucken des zweiten Teilstücks 12 für mehrere ophthalmologische Vorrichtungen 10 gleichzeitig erfolgt. Hierfür werden mehrere erste Teilstücke 11 gleichzeitig in der tomographischen Druckeinrichtung 8 angeordnet, wie dies beispielhaft bereits in der Fig. 2 gezeigt ist.

Es kann vorgesehen sein, dass eine Position des ersten Teilstücks 11 in der aushärtbaren Flüssigkeit 3 mittels mindestens einer von außen in die aushärtbare Flüssigkeit 3 eingespeisten Energie und/oder Kraft stabilisiert und/oder korrigiert wird. Hierzu weist die Anordnung 1 eine Positioniereinrichtung 14-x auf, welche das Stabilisieren und/oder Korrigieren durchführt. Die Positioniereinrichtung 14-x kann in der tomographischen Druckeinrichtung 8 angeordnet sein, solange diese den tomographischen Druckvorgang nicht beeinträchtigt und/oder an einer anderen Position am transparenten Rohr 2.

Es kann vorgesehen sein, dass eine Position des ersten Teilstücks 11 in der aushärtbaren Flüssigkeit 3 mittels mindestens einer optischen Pinzette 14-1 stabilisiert und/oder korrigiert wird.

Ferner kann alternativ oder zusätzlich vorgesehen sein, dass eine Position des ersten Teilstücks 11 in der aushärtbaren Flüssigkeit 3 mittels mindestens eines elektrischen Feldes und/oder mindestens eines magnetischen Feldes stabilisiert und/oder korrigiert wird, wobei die Positioniereinrichtung 14-x hierzu als Felderzeugungseinrichtung 14-2 ausgebildet ist, welche insbesondere ein gerichtetes und/oder fokussiertes elektrisches und/oder magnetisches Feld erzeugen kann.

Es kann alternativ oder zusätzlich vorgesehen sein, dass eine Position des ersten Teilstücks 11 in der aushärtbaren Flüssigkeit 3 mittels akusto-mechanischen Kräften stabilisiert und/oder korrigiert wird. Die Positioniereinrichtung 14-x ist dann als akusto-mechanischer Modulator 14-3 ausgebildet.

Es kann vorgesehen sein, dass eine Position des ersten Teilstücks 11 in der aushärtbaren Flüssigkeit 3 mittels einer entgegen der Schwerkraft ausgerichteten laminaren Strömung 4 stabilisiert und/oder korrigiert wird. Hierzu kann beispielsweise ein Strömungswinkel geändert werden. Das Rohr 2 kann entsprechende Leitelemente (nicht gezeigt) aufweisen, die zum Erzeugen bzw. Führen einer solchen laminaren Strömung 4 dienen.

Es kann grundsätzlich vorgesehen sein, dass die ophthalmologische Vorrichtung 10 mehrere erste Teilstücke 11 und/oder mehrere zweite Teilstücke 12 aufweist. Es kann insbesondere vorgesehen sein, dass weitere tomographische Druckeinrichtungen vorgesehen sind, um mehrere ophthalmologische Vorrichtungen 10 gleichzeitig herzustellen und/oder um weitere zweite Teilbereiche separat tomographisch zu drucken.

### Bezugszeichenliste

- 1: Anordnung
- 2: transparentes Rohr
- 3: aushärtbare Flüssigkeit
- 4: laminare Strömung
- 5: Strömungserzeugungseinrichtung
- 6: elektromagnetische Strahlung
- 7: Datenverarbeitungseinrichtung
- 8: tomographische Druckeinrichtung
- 8-1: Belichtungseinrichtung
- 9-1: Schleuse
- 9-2: Schleuse
- 10: ophthalmologische Vorrichtung
- 11: erstes Teilstück
- 12: zweites Teilstück
- 13: Kühleinrichtung
- 14-x: Positioniereinrichtung
- 14-1: optische Pinzette
- 14-2: Felderzeugungseinrichtung
- 14-3: akusto-mechanischer Modulator
- 15: Befülleinrichtung
- 16: Dreheinrichtung
- 20: Datensatz
- 21: Bilder
- 100-106: Verfahrensschritte

## Patentansprüche

1. Verfahren zum Herstellen einer ophthalmologischen Vorrichtung (10), umfassend:
Bereitstellen eines für elektromagnetische Strahlung (6) transparenten Rohres (2), Befüllen des transparenten Rohres (2) mit einer mittels elektromagnetischer Strahlung (6) aushärtbaren Flüssigkeit (3),
Einbringen von mindestens einem ersten Teilstück (11) der ophthalmologischen Vorrichtung (10) in das transparente Rohr (2),
Zuführen des mindestens einen ersten Teilstücks (11) mittels einer laminaren Strömung (4) in der aushärtbaren Flüssigkeit (3) zu mindestens einer tomographischen Druckeinrichtung (8),
Erzeugen und/oder Bereitstellen eines Datensatzes (20) aus Bildern (21) mindestens eines zweiten Teilstücks (12) der ophthalmologischen Vorrichtung (1), wobei die Bilder (21) Projektionen des mindestens einen zweiten Teilstücks (12) aus unterschiedlichen Richtungen beinhalten,
tomographisches Drucken der aushärtbaren Flüssigkeit (3) in dem transparenten Rohr (2) mittels elektromagnetischer Strahlung (6) ausgehend von dem erzeugten und/oder bereitgestellten Datensatz (20) zum Ausbilden des mindestens einen zweiten Teilstücks (12) mittels der mindestens einen tomographischen Druckeinrichtung (8),
wobei das Erzeugen und/oder Bereitstellen des Datensatzes (20) und das tomographische Drucken derart erfolgen, dass das mindestens eine zweite Teilstück (12) an dem mindestens einen ersten Teilstück (11) angeordnet ist und/oder dieses zumindest teilweise umschließt, und
Abführen des mindestens einen ersten Teilstücks (11) und des hieran angeordneten mindestens einen zweiten Teilstücks (12) mittels einer laminaren Strömung (4) in der aushärtbaren Flüssigkeit (3) von der mindestens einen tomographischen Druckeinrichtung (8).

2. Verfahren nach Anspruch 1, **dadurch gekennzeichnet, dass** die aushärtbare Flüssigkeit (3) einen vorgegebenen Anteil an Kieselgel umfasst.

3. Verfahren nach Anspruch 1 oder 2, **dadurch gekennzeichnet, dass** die aushärtbare Flüssigkeit (3) vorpolymerisiertes Methylmethacrylat und/oder eine Mischung aus langkettigen Polymeren und kurzen Monomeren umfasst.

4. Verfahren nach einem der vorangegangenen Ansprüche, **dadurch gekennzeichnet, dass** das transparente Rohr (2) zusammen mit der hierin enthaltenen aushärtbaren Flüssigkeit (3) zumindest während des tomographischen Druckens um eine Längsachse gedreht wird.

5. Verfahren nach einem der vorangegangenen Ansprüche, **dadurch gekennzeichnet, dass** die aushärtbare Flüssigkeit (3) zumindest abschnittsweise mittels einer Kühleinrichtung (13) gekühlt wird.

6. Verfahren nach einem der vorangegangenen Ansprüche, **dadurch gekennzeichnet, dass** das tomographische Drucken des jeweiligen mindestens einen zweiten Teilstücks (12) für mehrere ophthalmologische Vorrichtungen (10) gleichzeitig erfolgt.

7. Verfahren nach einem der vorangegangenen Ansprüche, **dadurch gekennzeichnet, dass** eine Position des mindestens einen ersten Teilstücks (11) in der aushärtbaren Flüssigkeit (3) mittels mindestens einer von außen in die aushärtbare Flüssigkeit (3) eingespeisten Energie und/oder Kraft stabilisiert und/oder korrigiert wird.

8. Verfahren nach Anspruch 7, **dadurch gekennzeichnet, dass** eine Position des mindestens einen ersten Teilstücks (11) in der aushärtbaren Flüssigkeit (3) mittels mindestens einer optischen Pinzette (14-1) stabilisiert und/oder korrigiert wird.

9. Verfahren nach Anspruch 7 oder 8, **dadurch gekennzeichnet, dass** eine Position des mindestens einen ersten Teilstücks (11) in der aushärtbaren Flüssigkeit (3) mittels mindestens eines elektrischen Feldes und/oder mindestens eines magnetischen Feldes stabilisiert und/oder korrigiert wird.

10. Verfahren nach einem der Ansprüche 7 bis 9, **dadurch gekennzeichnet, dass** eine Position des mindestens einen ersten Teilstücks (11) in der aushärtbaren Flüssigkeit (3) mittels akusto-mechanischen Kräften stabilisiert und/oder korrigiert wird.

11. Verfahren nach einem der vorangegangenen Ansprüche, **dadurch gekennzeichnet, dass** eine Position des mindestens einen ersten Teilstücks (11) in der aushärtbaren Flüssigkeit (3) mittels einer entgegen der Schwerkraft ausgerichteten laminaren Strömung stabilisiert und/oder korrigiert wird.

12. Anordnung (1) zum Herstellen einer ophthalmologischen Vorrichtung (10), umfassend:
ein für elektromagnetische Strahlung (6) transparentes Rohr (2),
eine Strömungserzeugungseinrichtung (5), die eingerichtet ist zum Erzeugen einer laminaren Strömung (4) in einer in dem transparenten Rohr (2) enthaltenen mittels elektromagnetischer Strahlung (6) aushärtbaren Flüssigkeit (3),
eine Datenverarbeitungseinrichtung (7), und
mindestens eine tomographische Druckeinrichtung (8),
wobei die Strömungserzeugungseinrichtung (5) ferner dazu eingerichtet ist und das transparente Rohr (2) derart angeordnet ist, mindestens ein erstes Teilstück (11) der ophthalmologischen Vorrichtung (10) mittels der erzeugten laminaren Strömung (4) zu der mindestens einen tomographischen Druckeinrichtung (8) zuzuführen und wieder von dieser abzuführen,
wobei die Datenverarbeitungseinrichtung (7) dazu eingerichtet ist, einen Datensatz (20) aus Bildern (21) mindestens eines zweiten Teilstücks (12) der ophthalmologischen Vorrichtung (10) zu erzeugen und/oder bereitzustellen, wobei die Bilder (21) Projektionen dieses mindestens einen zweiten Teilstücks (12) aus unterschiedlichen Richtungen beinhalten,
wobei die tomographische Druckeinrichtung (8) dazu eingerichtet ist, die aushärtbare Flüssigkeit (3) in dem transparenten Rohr (2) mittels elektromagnetischer Strahlung (6) ausgehend von dem erzeugten und/oder bereitgestellten Datensatz (20) zum Ausbilden des mindestens einen zweiten Teilstücks (12) tomographisch zu drucken,
wobei das Erzeugen und/oder Bereitstellen des Datensatzes (20) und das tomographische Drucken derart erfolgen, dass das mindestens eine zweite Teilstück (12) an dem mindestens einen ersten Teilstück (11) angeordnet ist und/oder dieses zumindest teilweise umschließt.

## Claims

1. Method for producing an ophthalmological device (10), comprising:
providing a tube (2) which is transparent to electromagnetic radiation (6),
filling the transparent tube (2) with a liquid (3) which is curable by means of electromagnetic radiation (6),
introducing at least one first component (11) of the ophthalmological device (10) into the transparent tube (2),
supplying the at least one first component (11) to at least one tomographic printing apparatus (8) by means of a laminar flow (4) in the curable liquid (3),
creating and/or providing a data record (20) made of images (21) of at least one second component (12) of the ophthalmological device (1), the images (21) containing projections of the at least one second component (12) from different directions,
tomographically printing the curable liquid (3) in the transparent tube (2) by means of electromagnetic radiation (6) using as starting point the created and/or provided data record (20) for forming the at least one second component (12) by means of the at least one tomographic printing apparatus (8),
the creation and/or provision of the data record (20) and the tomographic printing being implemented in such a way that the at least one second component (12) is arranged on the at least one first component (11) and/or at least partially encloses the latter, and
removing the at least one first component (11) and the at least one second component (12) arranged thereon from the at least one tomographic printing apparatus (8) by means of a laminar flow (4) in the curable liquid (3).

2. Method according to Claim 1, **characterized in that** the curable liquid (3) comprises a specified proportion of silica gel.

3. Method according to Claim 1 or 2, **characterized in that** the curable liquid (3) comprises prepolymerized methyl methacrylate and/or a mixture of long-chain polymers and short monomers.

4. Method according to any of the preceding claims, **characterized in that** the transparent tube (2) is rotated about a longitudinal axis together with the curable liquid (3) contained therein, at least during the tomographic printing.

5. Method according to any of the preceding claims, **characterized in that** the curable liquid (3) is cooled by means of a cooling apparatus (13), at least in sections.

6. Method according to any of the preceding claims, **characterized in that** tomographic printing of the respective at least one second component (12) is implemented simultaneously for a plurality of ophthalmological devices (10).

7. Method according to any of the preceding claims, **characterized in that** a position of the at least one first component (11) in the curable liquid (3) is stabilized and/or corrected by means of at least one energy and/or force applied to the curable liquid (3) from the outside.

8. Method according to Claim 7, **characterized in that** a position of the at least one first component (11) in the curable liquid (3) is stabilized and/or corrected by means of at least one optical tweezer (14-1).

9. Method according to Claim 7 or 8, **characterized in that** a position of the at least one first component (11) in the curable liquid (3) is stabilized and/or corrected by means of at least one electric field and/or at least one magnetic field.

10. Method according to any of Claims 7 to 9, **characterized in that** a position of the at least one first component (11) in the curable liquid (3) is stabilized and/or corrected by means of acoustomechanical forces.

11. Method according to any of the preceding claims, **characterized in that** a position of the at least one first component (11) in the curable liquid (3) is stabilized and/or corrected by means of a laminar flow aligned counter to the gravitational force.

12. Arrangement (1) for producing an ophthalmological device (10), comprising:
a tube (2) which is transparent to electromagnetic radiation (6),
a flow creation apparatus (5) configured to create a laminar flow (4) in a liquid (3) which is curable by means of electromagnetic radiation (6) and contained in the transparent tube (2),
a data processing apparatus (7), and
at least one tomographic printing apparatus (8),
the flow creation apparatus (5) further being configured and the transparent tube (2) being arranged to supply at least one first component (11) of the ophthalmological device (10) to, and remove said at least one first component from, the at least one tomographic printing apparatus (8) by means of the created laminar flow (4),
the data processing apparatus (7) being configured to create and/or provide a data record (20) made of images (21) of at least one second component (12) of the ophthalmological device (10), the images (21) containing projections of this at least one second component (12) from different directions,
the tomographic printing apparatus (8) being configured to tomographically print the curable liquid (3) in the transparent tube (2) by means of electromagnetic radiation (6) using as starting point the created and/or provided data record (20) for forming the at least one second component (12),
the creation and/or provision of the data record (20) and the tomographic printing being implemented in such a way that the at least one second component (12) is arranged on the at least one first component (11) and/or at least partially encloses the latter.

## Revendications

1. Procédé de fabrication d'un dispositif ophtalmologique (10), comprenant :
la fourniture d'un tube transparent (2) au rayonnement électromagnétique (6),
le remplissage du tube transparent (2) avec un liquide durcissable (3) au moyen d'un rayonnement électromagnétique (6),
l'introduction d'au moins une première section (11) du dispositif ophtalmologique (10) dans le tube transparent (2),
l'acheminement de ladite au moins une première section (11) au moyen d'un flux laminaire (4) dans le liquide durcissable (3) vers au moins un dispositif d'impression tomographique (8),
la génération et/ou la fourniture d'un ensemble de données (20) à partir d'images (21) d'au moins une deuxième section (12) du dispositif ophtalmologique (1), les images (21) contenant des projections de ladite au moins une deuxième section (12) à partir de différentes directions,
l'impression tomographique du liquide durcissable (3) dans le tube transparent (2) au moyen du rayonnement électromagnétique (6) à partir de l'ensemble de données (20) généré et/ou fourni pour former ladite au moins une deuxième section (12) au moyen dudit au moins un dispositif d'impression tomographique (8),
la génération et/ou la fourniture de l'ensemble de données (20) et l'impression tomographique étant effectuées de telle sorte que ladite au moins une deuxième section (12) soit agencée sur ladite au moins une première section (11) et/ou l'entoure au moins partiellement, et
l'expulsion de ladite au moins une première section (11) et de ladite au moins une deuxième section (12) qui y est agencée au moyen d'un flux laminaire (4) dans le liquide durcissable (3) dudit au moins un dispositif d'impression tomographique (8).

2. Procédé selon la revendication 1, **caractérisé en ce que** le liquide durcissable (3) comprend une proportion prédéfinie de gel de silice.

3. Procédé selon la revendication 1 ou 2, **caractérisé en ce que** le liquide durcissable (3) comprend du méthacrylate de méthyle prépolymérisé et/ou un mélange de polymères à chaîne longue et de monomères courts.

4. Procédé selon l'une quelconque des revendications précédentes, **caractérisé en ce que** le tube transparent (2) est amené à tourner autour d'un axe longitudinal avec le liquide durcissable (3) qu'il contient, au moins pendant l'impression tomographique.

5. Procédé selon l'une quelconque des revendications précédentes, **caractérisé en ce que** le liquide durcissable (3) est refroidi au moins par sections au moyen d'un dispositif de refroidissement (13).

6. Procédé selon l'une quelconque des revendications précédentes, **caractérisé en ce que** l'impression tomographique de ladite au moins une deuxième section (12) respective est effectuée simultanément pour plusieurs dispositifs ophtalmologiques (10).

7. Procédé selon l'une quelconque des revendications précédentes, **caractérisé en ce qu'**une position de ladite au moins une première section (11) dans le liquide durcissable (3) est stabilisée et/ou corrigée au moyen d'au moins une énergie et/ou force introduite de l'extérieur dans le liquide durcissable (3).

8. Procédé selon la revendication 7, **caractérisé en ce qu'**une position de ladite au moins une première section (11) dans le liquide durcissable (3) est stabilisée et/ou corrigée au moyen d'au moins une pince optique (14-1).

9. Procédé selon la revendication 7 ou 8, **caractérisé en ce qu'**une position de ladite au moins une première section (11) dans le liquide durcissable (3) est stabilisée et/ou corrigée au moyen d'au moins un champ électrique et/ou d'au moins un champ magnétique.

10. Procédé selon l'une quelconque des revendications 7 à 9, **caractérisé en ce qu'**une position de ladite au moins une première section (11) dans le liquide durcissable (3) est stabilisée et/ou corrigée au moyen de forces acoustomécaniques.

11. Procédé selon l'une quelconque des revendications précédentes, **caractérisé en ce qu'**une position de ladite au moins une première section (11) dans le liquide durcissable (3) est stabilisée et/ou corrigée au moyen d'un flux laminaire orienté en sens opposé à la gravité.

12. Agencement (1) pour la fabrication d'un dispositif ophtalmologique (10), comprenant :
un tube transparent (2) au rayonnement électromagnétique (6),
un dispositif de génération de flux (5), qui est conçu pour générer un flux laminaire (4) dans un liquide durcissable (3) contenu dans le tube transparent (2) au moyen du rayonnement électromagnétique (6),
un dispositif de traitement de données (7), et
au moins un dispositif d'impression tomographique (8),
le dispositif de génération de flux (5) étant en outre conçu et le tube transparent (2) étant agencé de manière à acheminer au moins une première section (11) du dispositif ophtalmologique (10) vers ledit au moins un dispositif d'impression tomographique (8) et à la ramener depuis celui-ci au moyen du flux laminaire (4) généré,
le dispositif de traitement de données (7) étant conçu pour générer et/ou fournir un ensemble de données (20) à partir d'images (21) d'au moins une deuxième section (12) du dispositif ophtalmologique (10), les images (21) contenant des projections de ladite au moins une deuxième section (12) à partir de différentes directions,
le dispositif d'impression tomographique (8) étant conçu pour imprimer par tomographie le liquide durcissable (3) dans le tube transparent (2) au moyen du rayonnement électromagnétique (6) à partir de l'ensemble de données (20) généré et/ou fourni pour former ladite au moins une deuxième section (12),
la génération et/ou la fourniture de l'ensemble de données (20) et l'impression tomographique étant effectuées de telle sorte que ladite au moins une deuxième section (12) soit agencée sur ladite au moins une première section (11) et/ou l'entoure au moins partiellement.
